# EUROPEAN PATENT SPECIFICATION

(11) **EP 2 644 163 B1**
(45) Date of publication and mention of the grant of the patent: **31.08.2016**
(21) Application number: 13157466.7
(22) Date of filing: 01.03.2013
(51) Int. Cl.: A61F 2/46

(54) **Instrument for removing an implanted humeral stem component**
Instrument zum Entfernen einer implantierten Humerusstammkomponente
Instrument pour l'élimination d'un composant de tige humérale implanté

(30) Priority: 29.03.2012 US 201213434304
(43) Date of publication of application: 02.10.2013
(73) Proprietor: DePuy Synthes Products, LLC, Raynham, MA 02767-0350 (US)
(72) Inventor: Chavarria, Jason M, Warsaw, IN Indiana 46581 (US); Lappin, Kyle E, Warsaw, IN Indiana 46581 (US); McElhaney, Patrick G Jr, Warsaw, IN Indiana 46581 (US)
(74) Representative: Belcher, Simon James

(56) References cited:
- EP-A1- 2 047 826
- EP-A1- 2 363 098
- DE-A1-102006 024 809
- FR-A1- 2 660 855

## Description

This invention relates generally to an orthopaedic instrument for use in the performance of an orthopaedic joint replacement procedure, and more particularly to an orthopaedic surgical instrument assembly for removing an implanted humeral stem EP 2047826 discloses an instrument according to the preamble of claim 1.

During the lifetime of a patient, it may be necessary to perform a total shoulder replacement procedure on the patient as a result of, for example, disease or trauma. In a total shoulder replacement procedure, a humeral prosthesis having a prosthetic head is used to replace the natural head of the patient's humerus. The humeral prosthesis typically includes an elongated humeral stem component that is implanted into the intramedullary canal of the patient's humerus. In such a total shoulder replacement procedure, the natural glenoid surface of the scapula is resurfaced or otherwise replaced with a glenoid component that provides a bearing surface upon which the prosthetic head of the humeral prosthesis articulates.

From time-to-time, revision surgery is performed to replace a previously-implanted humeral stem component. In such a revision surgery, the previously implanted humeral stem component is surgically removed and a replacement humeral stem component is implanted in the patient's humerus.

The present invention provides an orthopaedic surgical instrument for extracting an implanted humeral stem component for a patient's humerus, which includes a surgical tamp. The surgical tamp includes an elongated shaft having a proximal end and an opposite, distal end. A strike plate is secured to the proximal end of the elongated shaft, with an engagement tip being secured to the distal end of the elongated shaft. The proximal end defines a first longitudinal axis, with the distal end defining a second longitudinal axis. The second axis is offset from and parallel to the first axis.

The elongated shaft of the surgical tamp may further include a mid-shaft section connected to and extending distally from the proximal end, a first elbow connected to the mid-shaft section, and a second elbow connected to both the first elbow and the distal end of the elongated shaft.

The surgical tamp may also include a grip positioned around the elongated shaft at a location proximate to the strike plate.

The engagement tip may define a third longitudinal axis which is orthogonal to both the first and second longitudinal axes.

The second axis is offset from the first axis in a direction that is orthogonal to both the first and second axes.

The engagement tip of the surgical tamp may have a slot formed therein.

The instrument provided by the invention can be used in a method of surgically removing an implanted humeral stem component from the humerus of a patient, which includes cutting a bone fragment from a medial surface of the humerus of the patient so as to expose an inferior surface of a collar of the humeral stem component. Such method does not form part of the invention. An engagement tip of a surgical tamp in then positioned contact with the inferior surface of the collar of the humeral stem component. The surgical tamp is then impacted so as to urge the humeral stem component from the humerus of the patient.

An osteotomy may be performed on the medial surface of the humerus of the patient to cut the bone fragment from the medial surface of the humerus of the patient. Such an osteotomy may be performed by advancing an osteotome into the medial surface of the humerus of the patient at a location under an inferior edge of the collar of the humeral stem component.

The surgical tamp may include a handle that is offset from the engagement tip. In such an embodiment, the handle of the surgical tamp is impacted along a first axis so as to apply an extraction force to the humeral stem component along a second axis. The first axis being parallel to, and spaced apart from, the second axis.

The engagement tip of the surgical tamp has a slot formed therein such that a medial fin of the humeral stem component is received into the slot.

The instrument provided by the invention can be used in a method of surgically removing an implanted humeral stem component from a humerus of a patient, which includes positioning an engagement tip of an offset surgical tamp in contact with an inferior surface of the collar of the humeral stem component. The surgical tamp may be impacted along a first axis so as to apply an extraction force to the humeral stem component along a second axis. The first axis is parallel to, and spaced apart from, the second axis.

In an illustrative example, the method may also include cutting a bone fragment from a medial surface of the humerus of the patient so as to expose the inferior surface of the collar of the humeral stem component. An osteotomy may be performed on the medial surface of the humerus of the patient to cut the bone fragment from the medial surface of the humerus of the patient. Such an osteotomy may be performed by advancing an osteotome into the medial surface of the humerus of the patient at a location under an inferior edge of the collar of the humeral stem component.

The surgical tamp may include a handle that is offset from the engagement tip. In such an embodiment, the handle of the surgical tamp is impacted along the first axis so as to apply the extraction force to the humeral stem component along the second axis. The engagement tip of the surgical tamp may have a slot formed therein such that a medial fin of the humeral stem component is received into the slot.

In some illustrative examples, positioning the engagement tip of the surgical tamp in contact with the inferior surface of the collar of the humeral stem component may include impacting the surgical tamp along a third axis extending orthogonal to a longitudinal axis of the humeral stem component.

Embodiments of the invention are described below by way of example with reference to the accompanying drawings, in which:
FIG. 1 is a perspective view of an orthopaedic surgical tamp that may be used in a surgical procedure to extract an implanted humeral stem component,
FIG. 2 is a cross-sectional view of the surgical tamp taken along the line 2-2 of FIG. 1, as viewed in the direction of the arrows,
FIG. 3 is a perspective view showing a humeral stem component implanted in the humerus of a patient,
FIG. 4 is a view similar to FIG. 3, but showing a bone fragment having been removed from a medial side of the patient's humerus so as to expose an inferior surface of the collar of the humeral stem component,
FIG. 5 is a fragmentary elevational view showing the surgical tamp engaged with the inferior surface of the collar of the humeral stem component,
FIG. 6 is an enlarged fragmentary perspective view showing the surgical tamp engaged with the inferior surface of the collar of the humeral stem component,
FIG. 7 is an enlarged fragmentary elevational view showing the surgical tamp engaged with the inferior surface of the collar of the humeral stem component, note that a portion of the humerus has been cutaway in the drawing of FIG. 7 for clarity of description,
FIG. 8 is a view similar to FIG. 6, but showing the humeral stem component being removed from the humerus of the patient,
FIG. 9 is a view similar to FIG. 7, but showing the humeral stem component being removed from the humerus of the patient,
FIG. 10 is an enlarged fragmentary perspective view showing another embodiment of an orthopaedic surgical tamp and the collar of the humeral stem component,
FIG. 11 is a fragmentary elevational view showing the surgical tamp of FIG. 10 impacted into the humerus of the patient,
FIG. 12 is a view similar to FIG. 10 showing the surgical tamp engaged with the inferior surface of the collar of the humeral stem component, and
FIG. 13 is a fragmentary elevational view showing the surgical tamp engaged with the inferior surface of the collar of the humeral stem component.

Terms representing anatomical references, such as anterior, posterior, medial, lateral, superior and inferior, may be used throughout this document in reference to both the orthopaedic implants described herein and a patient's natural anatomy. Such terms have well-understood meanings in both the study of anatomy and the field of orthopaedics. Use of such anatomical reference terms in the document is intended to be consistent with their well-understood meanings unless noted otherwise.

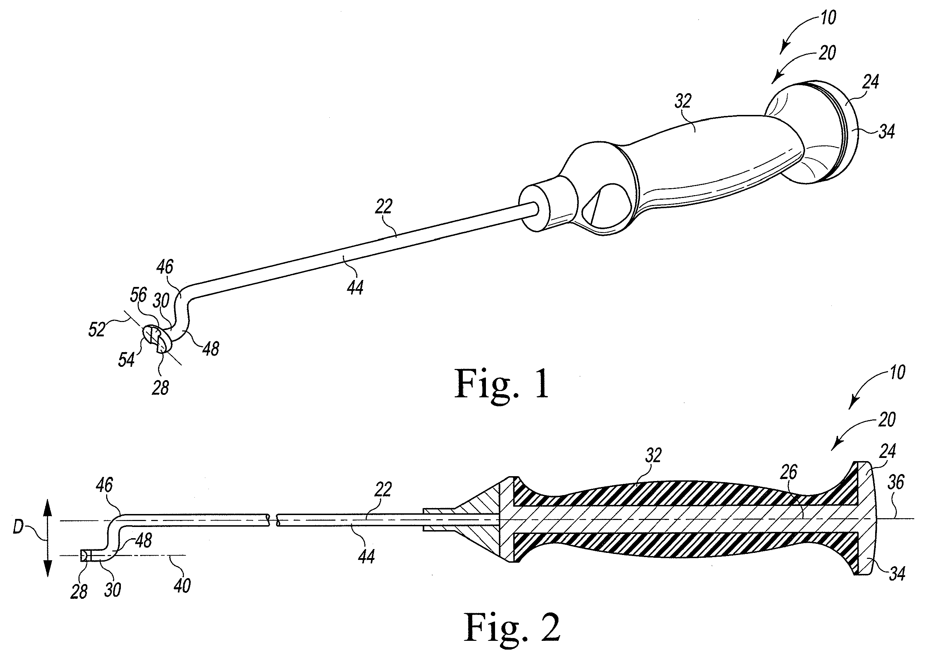

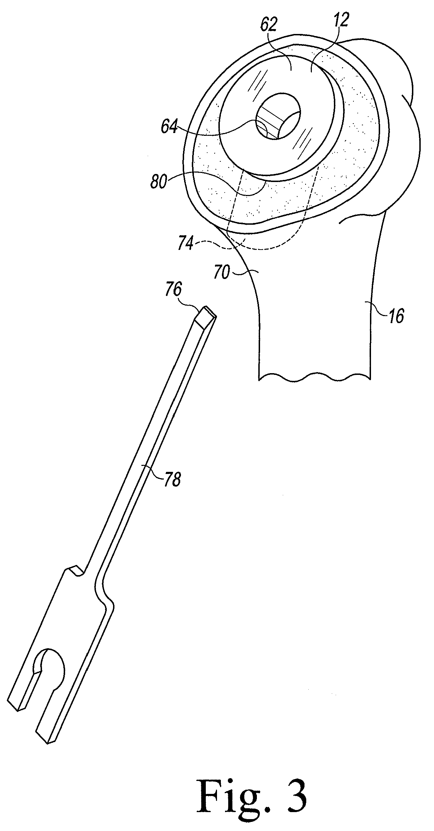

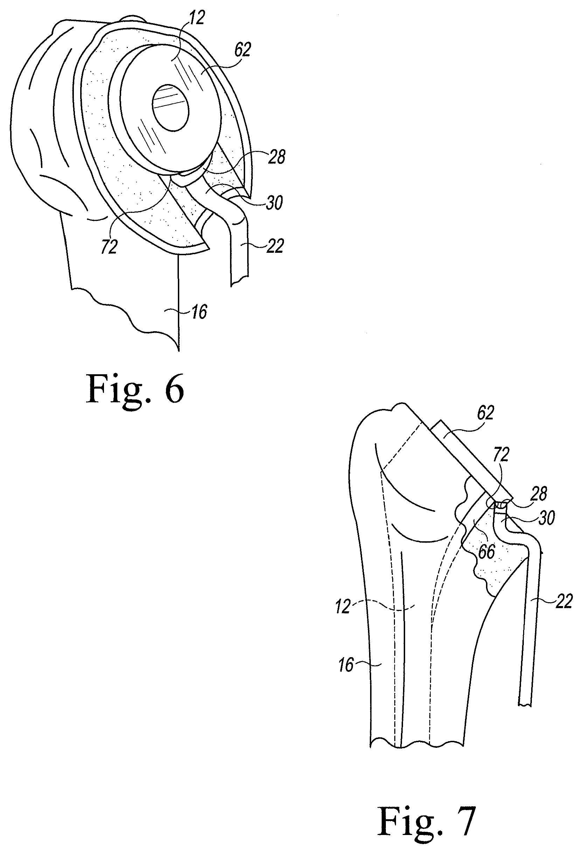

Referring to the drawings, FIGS. 1 and 2 show an orthopaedic surgical instrument 10 for removing an implanted humeral stem component 12 from the intramedullary canal 14 of the humerus 16 of a patient (see also FIGS. 3 to 9). The surgical instrument 10 is embodied as an offset surgical tamp 20. The offset surgical tamp 20 includes an elongated shaft 22 having an impact head 24 on its proximal end 26 and an engagement tip 28 on its distal end 30. A sleeve or grip 32 is positioned around, and immovably coupled to, the outer surface of the surgical tamp's shaft 22 such as by, for example, overmoulding. The sleeve 32 functions as a grip for allowing the surgeon to hold the offset surgical tamp 20 during a surgical procedure to extract the humeral stem component 12 from the patient's humerus 16.

The impact head 24 of the offset surgical tamp 20 includes a circular metal strike plate 34. In use, the surgeon holds the offset surgical tamp 20 using the grip 32 and strikes the metal strike plate 34 with a surgical mallet, sledge, or other impaction tool to generate an extraction force to extract the humeral stem component 12 from the patient's humerus 16.

As can be seen best in FIG. 2, the proximal end 26 of the surgical tamp's elongated shaft 22 is offset from its distal end 30. In particular, the proximal end 26 of the elongated shaft 22 has a longitudinal axis 36, with the distal end 30 of the shaft having a different longitudinal axis 40 that is offset from, and parallel to, the longitudinal axis 36 of the surgical tamp's proximal end 26. In the embodiment shown in the drawings, the longitudinal axis 40 of the distal end 30 of the shaft 22 is offset from the longitudinal axis 36 of the surgical tamp's proximal end 26 in a direction D that is orthogonal to both of the axes 36, 40.

Such an offset geometry is accomplished by a pair of 90° elbows formed in the elongated shaft 22. In particular, the proximal end 26 of the shaft 22 transitions to a mid-shaft section 44 that extends distally away from the proximal end 26 and, in turn, transitions to an elbow 46. The elbow 46 extends orthogonally away from the mid-shaft section 44 and transitions to another elbow 48. The elbow 48, in turn transitions to the distal end 30 of the elongated shaft 22.

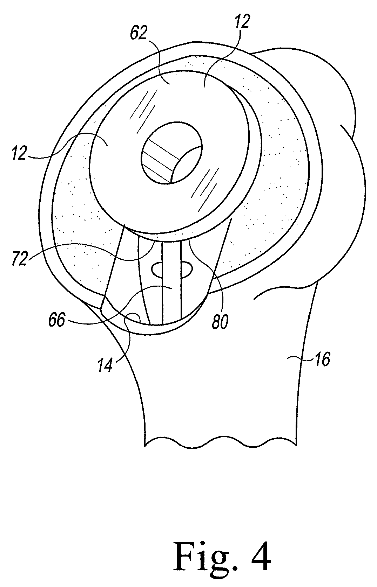

As can be seen in FIG. 1, the engagement tip 28 is arcuate in shape and extends in a direction that is orthogonal to the distal end 30 of the elongated shaft 22. In particular, the engagement tip has a longitudinal axis 52 that is orthogonal to the longitudinal axis 40 of the distal end 30 of the shaft 22. The engagement tip 28 has a generally planar engaging face 54 having a slot 56 formed therein. The slot 56 allows the engagement tip 28 to straddle a medial fin 66 of the humeral stem component 12 (see FIG. 4).

The metallic components of offset surgical tamp 20 (e.g. the elongate shaft 22 and the strike plate 34) may be constructed from a medical-grade metal such as stainless steel, cobalt chromium alloy, or titanium, although other metals or alloys may be used. Moreover, in some embodiments, rigid polymers such as polyaryetheretherketone (PEEK) may also be used. The grip 32 may be constructed from a polymer such as silicone.

FIGS. 3 to 9 show a revision surgical procedure in which the surgical instrument 10 is used to surgically extract (i.e. remove) a previously implanted humeral stem component 12 from the intramedullary canal 14 of the humerus 16 of a patient. As can be seen in FIG. 3, in a previous primary procedure, the humeral stem component 12 was implanted in the patient's humerus 16. A typical humeral stem component 12 includes an annular-shaped collar 62 having a tapered bore 64 formed therein to receive a tapered stem of spherical head component (not shown). The collar 62 is positioned on a substantially planar surgically-resected humeral surface formed during the primary procedure. The collar 62 is formed on the superior surface of the body of the humeral stem component 12. A medial fin 66 extends along the medial surface of the humeral stem component 12, as shown in FIG. 4.

During a revision surgery, it may be necessary to remove the previously implanted humeral stem component 12 from the patient's humerus 16. As shown in FIGS. 3 to 6, the offset surgical tamp 20 may used to surgically remove a previously implanted humeral stem component 12.

To do so, a bone fragment is first cut from a medial surface 70 of the humerus 16 of the patient so as to expose an inferior surface 72 of the collar 62 of the implanted humeral stem component 12. As shown in FIG. 3, one way to cut the bone in such a manner is to perform an osteotomy on the medial surface 70 of the humerus 16 of the patient so as to cut the bone fragment 74 (shown in phantom in FIG. 3 prior to removal) from the medial surface 70 of the humerus 16. To do so, the cutting tip 76 of an osteotome 78 is advanced into the medial surface 70 of the humerus 16 of the patient at a location under an inferior edge 80 of the collar 62 of the humeral stem component 12. The motion of the cutting tip 76 causes the bone fragment 74 to be cut and thereafter removed. The bone fragment 74 may be saved for re-implantation later in the surgical procedure.

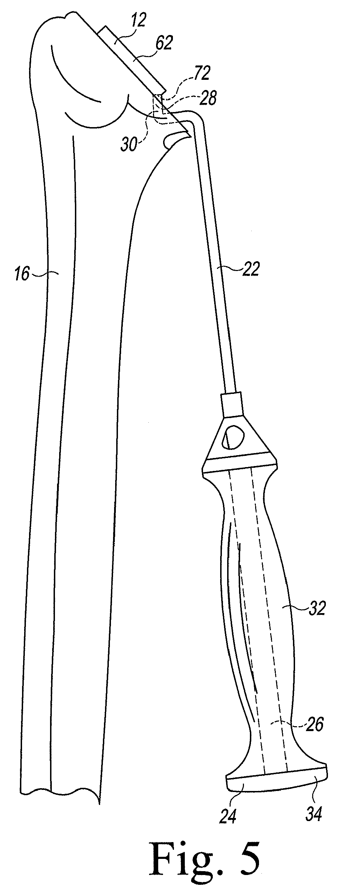

Once the inferior surface 72 of the collar 62 of the implanted humeral stem component 12 has been exposed, the surgeon may then use the offset surgical tamp 20 to extract the humeral stem component 12. To do so, as shown in FIGS. 5 to 7, the surgeon first advances the surgical tamp 20 such that its engagement tip 28 is positioned in contact with the inferior surface 72 of the collar 62 of the implanted humeral stem component 12. During such positioning of the engagement tip 28, the medial fin 66 of the humeral stem component 12 is received into the slot 56 thereby allowing the engagement tip 28 to straddle a medial fin 66 and firmly engage the inferior surface 72 of the collar 62.

Once the engagement tip 28 is positioned against the inferior surface 72 of the collar 62 in such a manner, the surgeon strikes the metal strike plate 34 with a surgical mallet, sledge, or other impaction tool to generate an extraction force that is applied to the inferior surface 72 of the collar 62. As can be seen in FIG. 5, because of the offset design of the offset surgical tamp 20 the extraction force is transmitted to the collar 62 along a different axis than as applied to the strike plate 34. In particular, the surgeon impacts the metal strike plate 34 along an axis that corresponds to the longitudinal axis 36 of the proximal end 26 of the elongated shaft 22. However, the resulting extraction force is applied to the inferior surface 72 of the collar 62 along an axis that corresponds to the longitudinal axis 40 of the distal end 30 of the shaft 22.

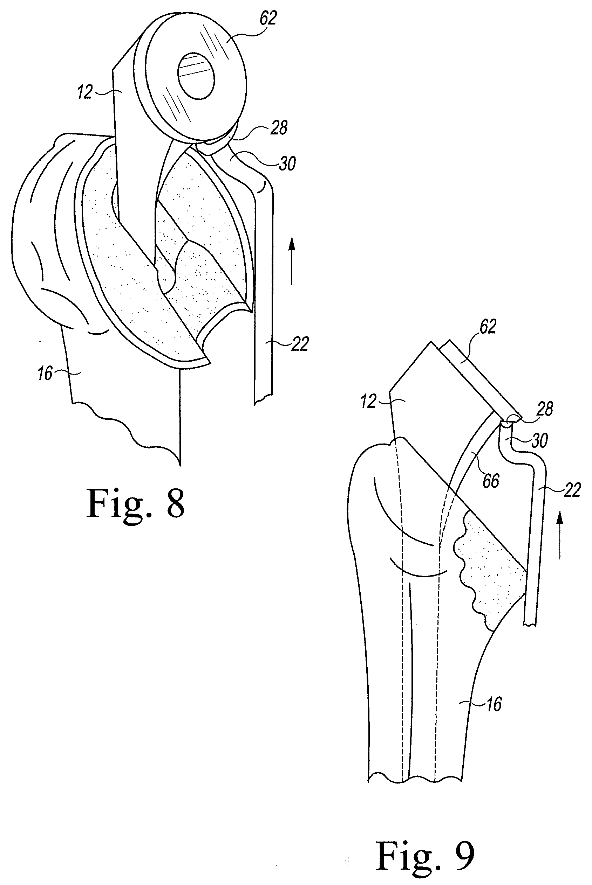

Repeated strikes from the surgeon onto the metal strike plate 34 of the handle urges the implanted humeral stem component 12 from the patient's humerus 16 as shown in FIGS. 8 and 9. The surgeon may then lift away the freed humeral stem component 12 from the patient's humerus 16. The surgeon may then perform the remainder of the revision surgical procedure, including the implantation of a revision humeral stem component (not shown) into the patient's humerus 16.

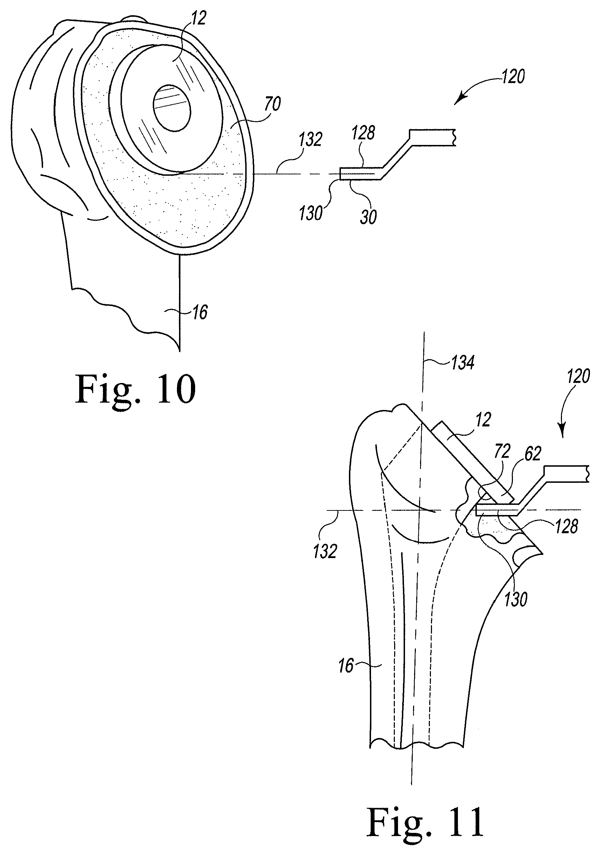

FIGS. 10 to 13 show another embodiment of an offset surgical tamp (hereinafter surgical tamp 120) with a modified engagement tip 128 formed on its distal end 30. As shown in FIG. 10, the engagement tip 128 includes a cutting edge 130 configured to engage with the substantially planar surgically-resected medial surface 70 of the patient's humerus 16. During a revision surgery, the engagement tip 128 of the surgical tamp 120 is impacted into the medial surface 70 along an axis 132 that extends orthogonal to a longitudinal axis 134 of the humeral stem component 12. In other words, the surgical tamp 120 is impacted perpendicular to the patient's humerus 16. As shown in FIG. 11, the cutting edge 130 cuts into the medial surface 70 of the humerus 16 to position the engagement tip 128 below the collar 62 of the implanted humeral stem 12.

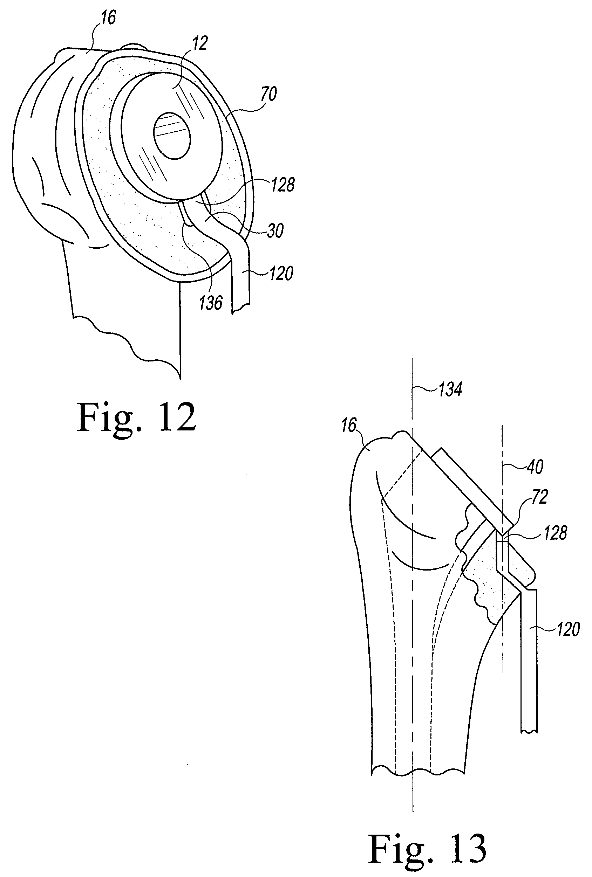

Once the engagement tip 128 is positioned below the collar 62, the surgeon may rotate the surgical tamp 120 downward to position the engagement tip 128 in contact with the inferior surface 72 of the collar 62 of the humeral stem component 12, as shown in FIGS. 12 and 13. As the surgical tamp 120 is rotated, the distal end 30 of the surgical tamp 120 is pressed into the medial surface 70, thereby forming a groove or depression 136 in the medial surface 70. When properly positioned, the longitudinal axis 40 of the surgical tamp 120 extends parallel to the longitudinal axis 134 of the humeral stem component 12 (i.e., parallel to the patient's humerus).

Once the engagement tip 128 is positioned against the inferior surface 72 of the collar 62 in such a manner, the surgeon strikes the metal strike plate 34 of the surgical tamp 120 with a surgical mallet, sledge, or other impaction tool to generate an extraction force that is applied to the inferior surface 72 of the collar 62 to urge the implanted humeral stem component 12 from the patient's humerus 16, as described above with reference to FIGS. 1 to 9. The surgeon may then lift away the freed humeral stem component 12 from the patient's humerus 16. The surgeon may then perform the remainder of the revision surgical procedure, including the implantation of a revision humeral stem component (not shown) into the patient's humerus 16.

An exemplary method of surgically removing an implanted humeral stem component from a humerus of a patient, using the instrument of the invention, can involve the steps of:
cutting a bone fragment from a medial surface of the humerus of the patient so as to expose an inferior surface of a collar of the humeral stem component,
positioning an engagement tip of a surgical tamp in contact with the inferior surface of the collar of the humeral stem component, and
impacting the surgical tamp so as to urge the humeral stem component from the humerus of the patient.

Optionally, the step of cutting the bone fragment involves performing an osteotomy on the medial surface of the humerus of the patient so as to cut the bone fragment from the medial surface of the humerus of the patient thereby exposing the inferior surface of the collar of the humeral stem component.

Optionally, the step of performing the osteotomy on the medial surface of the humerus of the patient comprises advancing an osteotome into the medial surface of the humerus of the patient at a location under an inferior edge of the collar of the humeral stem component.

Optionally, the surgical tamp comprises a handle that is offset from the engagement tip, and impacting the surgical tamp comprises impacting the handle of the surgical tamp so as to urge the humeral stem component from the humerus of the patient.

Optionally, the step of impacting the surgical tamp comprises impacting the handle of the surgical tamp along a first axis so as to apply an extraction force to the humeral stem component along a second axis, the first axis being parallel to, and spaced apart from, the second axis.

Optionally, the engagement tip of the surgical tamp has a slot formed therein, and the step of positioning the engagement tip of the surgical tamp in contact with the inferior surface of the collar of the humeral stem component comprises positioning the engagement tip of the surgical tamp such that a medial fin of the humeral stem component is received into the slot.

An exemplary method of surgically removing an implanted humeral stem component from a humerus of a patient, using the instrument of the invention, can involve the steps of:
positioning an engagement tip of an offset surgical tamp in contact with an inferior surface of a collar of the humeral stem component, and
impacting the surgical tamp along a first axis so as to apply an extraction force to the humeral stem component along a second axis, the first axis being parallel to, and spaced apart from, the second axis.

Optionally, the method can include the step of cutting a bone fragment from a medial surface of the humerus of the patient so as to expose the inferior surface of the collar of the humeral stem component.

Optionally, the step of cutting the bone fragment involves performing an osteotomy on the medial surface of the humerus of the patient so as to cut the bone fragment from the medial surface of the humerus of the patient thereby exposing the inferior surface of the collar of the humeral stem component.

Optionally, the step of performing the osteotomy on the medial surface of the humerus of the patient comprises advancing an osteotome into the medial surface of the humerus of the patient at a location under an inferior edge of the collar of the humeral stem component.

Optionally, the engagement tip of the surgical tamp has a slot formed therein, and the step of positioning the engagement tip of the surgical tamp in contact with the inferior surface of the collar of the humeral stem component comprises positioning the engagement tip of the surgical tamp such that a medial fin of the humeral stem component is received into the slot.

Optionally, the surgical tamp comprises a handle that is offset from the engagement tip, and the step of impacting the surgical tamp comprises impacting the handle of the surgical tamp so as to urge the humeral stem component from the humerus of the patient.

Optionally, the step of impacting the surgical tamp comprises impacting the handle of the surgical tamp along the first axis so as to apply an extraction force to the humeral stem component along the second axis.

Optionally, the step of positioning the engagement tip of the surgical tamp in contact with the inferior surface of the collar of the humeral stem component comprises impacting the surgical tamp along a third axis extending orthogonal to a longitudinal axis of the humeral stem component.

## Claims

1. An orthopaedic surgical instrument (10) comprising a surgical tamp (20) which comprises:
(i) an elongated shaft (22) having a proximal end (26) and an opposite, distal end (30),
(ii) a strike plate (34) secured to the proximal end of the elongated shaft, and
(iii) an engagement tip (28) secured to a distal end of the elongated shaft,
in which:
(a) the proximal end defines a first longitudinal axis (36),
(b) the distal end defines a second longitudinal axis (40), and
(c) the second axis is offset from and parallel to the first axis,
**characterised in that** the instrument is for extracting an implanted humeral stem component for a patient's humerus, the humeral stem component having a collar and a medial fin, the fin being adjacent to the collar, and the engagement tip of the surgical tamp has a slot (56) formed in it in which the medial fin on the implanted humeral stem component can be received when the engagement tip is positioned in contact with the inferior surface of the collar on the humeral stem component.

2. The orthopaedic surgical instrument of claim 1, in which the elongated shaft (22) of the surgical tamp (20) further includes: (i) a mid-shaft section (44) connected to and extending distally from the proximal end (26), (ii) a first elbow (46) connected to the mid-shaft section, (iii) a second elbow (48) connected to both the first elbow and the distal end of the elongated shaft.

3. The orthopaedic surgical instrument of claim 1, in which the surgical tamp (20) further comprises a grip (32) positioned around the elongated shaft (22) at a location proximate to the strike plate (34).

4. The orthopaedic surgical instrument of claim 1, in which the engagement tip (28) defines a third longitudinal axis, and the third longitudinal axis is orthogonal to both the first and second longitudinal axes (36, 40).

5. The orthopaedic surgical instrument of claim 1, in which the second axis (40 is offset from the first axis (36) in a direction that is orthogonal to both the first and second axes.

6. A kit for use in the performance of a revision surgical procedure on a patient's humerus in which the humerus has implanted in it a humeral stem component (12) having a collar (62) and a medial fin (66) adjacent to the collar, the kit comprising the said humeral stem component and an orthopaedic surgical instrument as claimed in any one of claims 1 to 5.

## Patentansprüche

1. Orthopädisches Operationsinstrument (10), umfassend ein Operationswerkzeug (20), das umfasst:
(i) einen länglichen Schaft (22) mit einem proximalen Ende (26) und einem entgegengesetzten bzw. gegenüberliegenden distalen Ende (30),
(ii) eine Anlageplatte (34), die an dem proximalen Ende des länglichen Schaftes gesichert ist, und
(iii) eine Eingriffsspitze (28), die an einem distalen Ende des länglichen Schaftes gesichert ist,
wobei
(a) das proximale Ende eine erste Längsachse (36) festlegt,
(b) das distale Ende eine zweite Längsachse (40) festlegt, und
(c) die zweite Achse von der ersten Achse versetzt und parallel zu dieser ist,
**dadurch gekennzeichnet, dass** das Instrument dem Extrahieren einer implantierten Humerus- bzw. Oberarmknochenschaftkomponente für den Humerus bzw. Oberarmknochen eines Patienten dient, wobei die Humerus- bzw. Oberarmknochenschaftkomponente einen Kragen und eine Zentralrippe aufweist, wobei die Rippe zu dem Kragen benachbart ist,
und die Eingriffsspitze des Operationswerkzeuges einen darin ausgebildeten Schlitz (56) aufweist, in dem die Zentralrippe an der implantierten Humerus- bzw. Oberarmknochenschaftkomponente aufgenommen werden kann, wenn die Eingriffsspitze in Kontakt mit der unteren Oberfläche des Kragens an der Humerus- bzw. Oberarmknochenschaftkomponente positioniert ist.

2. Orthopädisches Operationsinstrument nach Anspruch 1, wobei der längliche Schaft (22) des Operationswerkzeuges (20) des Weiteren beinhaltet: (i) einen Zentralschaftabschnitt (44), der mit dem proximalen Ende (26) verbunden ist und sich distal von diesem aus erstreckt, (ii) eine erste Biegung (46), die mit dem Zentralschaftabschnitt verbunden ist, (iii) eine zweite Biegung (48), die sowohl mit der ersten Biegung wie auch dem distalen Ende des länglichen Schaftes verbunden ist.

3. Orthopädisches Operationsinstrument nach Anspruch 1, wobei das Operationswerkzeug (20) des Weiteren einen Griff (32) umfasst, der um den länglichen Schaft (22) herum an einer Stelle nahe an der Anlageplatte (34) positioniert ist.

4. Orthopädisches Operationsinstrument nach Anspruch 1, wobei die Eingriffsspitze (28) eine dritte Längsachse festlegt und die dritte Längsachse senkrecht sowohl zu der ersten wie auch der zweiten Längsachse (36, 40) ist.

5. Orthopädisches Operationsinstrument nach Anspruch 1, wobei die zweite Achse (40) von der ersten Achse (36) in einer Richtung versetzt ist, die senkrecht sowohl zu der ersten wie auch der zweiten Achse ist.

6. Einrichtung zur Verwendung bei der Durchführung einer Prüf- bzw. Korrekturoperationsmaßnahme am Humerus bzw. Oberarmknochen eines Patienten, wobei in dem Humerus bzw. Oberarmknochen eine Humerus- bzw. Oberarmknochenschaftkomponente (12) implantiert ist, die einen Kragen (62) und eine zu dem Kragen benachbarte Zentralrippe (66) aufweist,
wobei die Einrichtung die Humerus- bzw. Oberarmknochenschaftkomponente und ein orthopädisches Operationsinstrument nach einem der Ansprüche 1 bis 5 umfasst.

## Revendications

1. Instrument chirurgical orthopédique (10) comprenant un dispositif de tassement chirurgical (20) qui comprend :
(i) un arbre allongé (22) ayant une extrémité proximale (26) et une extrémité distale (30) opposée,
(ii) une plaque de butée (34) fixée sur l'extrémité proximale de l'arbre allongé, et
(iii) une pointe de mise en prise (28) fixée sur une extrémité distale de l'arbre allongé,
dans lequel :
(a) l'extrémité proximale définit un premier axe longitudinal (36),
(b) l'extrémité distale définit un deuxième axe longitudinal (40), et
(c) le deuxième axe est décalé de et parallèle au premier axe,
**caractérisé en ce que** l'instrument est prévu pour extraire un composant de tige humérale implanté pour l'humérus d'un patient, le composant de tige humérale ayant un collier et une ailette médiale, l'ailette étant adjacente au collier,
et la pointe de mise en prise du dispositif de tassement chirurgical a une fente (56) formée dans cette dernière, dans laquelle l'ailette médiale sur le composant de tige humérale implanté peut être reçue lorsque la pointe de mise en prise est positionnée en contact avec la surface inférieure du collier sur le composant de tige humérale.

2. Instrument chirurgical orthopédique selon la revendication 1, dans lequel la tige allongée (22) du dispositif de tassement chirurgical (20) comprend en outre : (i) une section d'arbre central (44) raccordée à et s'étendant de manière distale à partir de l'extrémité proximale (26), (ii) une premier coude (46) raccordé à la section d'arbre central, (iii) un second coude (48) raccordé à la fois au premier coude et à l'extrémité distale de l'arbre allongé.

3. Instrument chirurgical orthopédique selon la revendication 1, dans lequel le dispositif de tassement chirurgical (20) comprend en outre une poignée (32) positionnée autour de l'arbre allongé (22) à un emplacement à proximité de la plaque de butée (34).

4. Instrument chirurgical orthopédique selon la revendication 1, dans lequel la pointe de mise en prise (28) définit un troisième axe longitudinal, et le troisième axe longitudinal est orthogonal à la fois aux premier et deuxième axes longitudinaux (36, 40).

5. Instrument chirurgical orthopédique selon la revendication 1, dans lequel le deuxième axe (40) est décalé du premier axe (36) dans une direction qui est orthogonale à la fois aux premier et deuxième axes.

6. Kit destiné à être utilisé pour réaliser une procédure chirurgicale de révision sur l'humérus d'un patient, dans lequel l'humérus a, implanté dans ce dernier, un composant de tige humérale (12) ayant un collier (62) et une ailette médiale (66) adjacente au collier, le kit comprenant ledit composant de tige humérale et un instrument chirurgical orthopédique selon l'une quelconque des revendications 1 à 5.
